# EUROPEAN PATENT SPECIFICATION

(11) **EP 1 424 127 B1**
(45) Date of publication and mention of the grant of the patent: **17.10.2007**
(21) Application number: 02764894.8
(22) Date of filing: 26.07.2002
(51) Int. Cl.: B01J 31/02, C07D 335/02

(54) **THIOPYRYL CATION AND DERIVATIVES OF SAME TRAPPED IN A ZEOLITIC MATERIAL**
THIOPYRYLKATION UND DERIVATE DAVON, EINGESCHLOSSEN IN EINEM ZEOLITHISCHEN MATERIAL
CATION THIOPIRYLE ET SES DERIVES NOYES DANS UNE MATIERE ZEOLITIQUE

(30) Priority: 27.07.2001 ES 200101839
(43) Date of publication of application: 02.06.2004
(73) Proprietor: UNIVERSIDAD POLITECNICA DE VALENCIA, 46022 Valencia (ES)
(72) Inventor: ALVARO RODRIGUEZ, Mercedes, E-46022 Valencia (ES); GARCIA GOMEZ, H., Univ. Politécnicade Valencia, E-46022 Valencia (ES); NARAYANA PILLAI, M., Univ. Politécnica de Valencia, E-46022 Valencia (ES)
(74) Representative: Armijo Navarro-Reverter, Enrique
(86) International application number: PCT/ES2002/000377
(87) International publication number: WO 2003/011456

(56) References cited:
- CORMA A. ET AL: 'Photoinduced electron transfer within zeolite cavities: cis-stilbene isomerization photosensitized by 2,4,6-triphenylpyrylium cation imprisoned inside zeolite Y' J. AM. CHEM. SOC. vol. 116, no. 6, 1994, pages 2276 - 2280, XP002051268
- CORMA A. ET AL: 'Highly efficient photoinduced electron transfer with 2,4,6-triphenylpyrylium cation incorporated inside extra large pore zeotype MCM-41' J. AM. CHEM. SOC. vol. 116, no. 21, 1994, pages 9767 - 9768, XP002976093
- CANO M.L. ET AL: 'Intrazeolite photochemistry. 13. Photophysical properties of bulky 2,4,6-triphenylpyrylium and tritylium cations with large- and extra-large pore zeolites' J. PHYS. CHEM. vol. 100, no. 46, 1996, pages 18152 - 18157, XP002976082
- SANJUAN A. ET AL: 'An organic sensitizer within Ti-zeolites as photocatalyst for the selective oxidation of olefins using oxygen and water' CHEM. COMMUN. vol. 17, 1999, pages 1641 - 1642, XP002976083
- WIZINGER R. ET AL: 'Thiopyrylium salts' HELV. CHIM. ACTA vol. 39, no. 22-23, 1956, pages 207 - 216, XP002976096
- JAYANTHI S.S. ET AL: 'Excited singlet state reactions of thiopyrylium with electron donors: electron transfer, induction of triplet by internal and external heavy atom effect and comparison of pyrylium and thiopyrylium reactions' J. PHYS. CHEM. A vol. 102, no. 3, 1998, pages 511 - 518, XP002976084
- PARRET S. ET AL: 'Fluorescence properties of pyrylium and thiopyrylium salts' BULL. CHEM. SOC. JAPAN vol. 68, no. 10, 1995, pages 2791 - 2795, XP002976094
- CORMA A. ET AL: 'Carbocations and organic radical cations inside zeolite matrices: generation, characterization, stability and properties' TOPICS IN CATALYSIS vol. 6, no. 1-4, 1998, pages 127 - 140, XP002976095

## Description

The present invention is encompassed within the field of zeolitic materials which comprise cations trapped therein.

### STATE OF THE ART

The thiopyrylium cation and its derivatives are photochemical sensitizers suitable for photochemically initiating, stimulating and catalyzing a large number of organic reactions. Among the latter are included dimerisation reactions, [2+2] and [2+4] cycloadditions including Diels-Alder reactions, isomerisation, oxidation, denitrogenation, and cleavages entailing the breakage of C-C bonds.

Thiopyrylium cations are also used in combination with peroxides, hydroperoxides, azoalkanes, halogenated compounds, etc., as polymerisation and crosslinking photoinitiators in processes such as photolithography, photopolymerisation, photocrosslinking and photocuring. In some of these processes, adequate sized particles of an inert material can be present, acting as a filler or charge providing a desired improvement in the mechanical properties such as hardness, resistance, etc.

The positive charge of these organic cations is generally compensated by simple inorganic anions such as perchlorate, tetrafluoroborate, halides, phosphorus hexafluoride, etc.

The different synthesis methodologies of these cations are described in the literature. In this way, a general means for obtaining these cations consists in the treatment of analogous pyrylium cations reacting with sulfide or bisulfide, as indicated below in diagram 1.

Zeolites are crystalline aluminium silicates of variable composition, the rigid structure of which defines perfectly regular channels and cavities called micropores. These inner spaces are accessible to organic molecules provided that the size of these is smaller than the dimensions of the micropores, and therefore, a mass transfer can occur from the outer part of the zeolite particles to the inner part of the micropores. Tridirectional large-pore zeolites also exist, which are those wherein the geometry of the inner spaces defines cavities arranged in the three dimensions of the crystal lattice, and which are interconnected by openings defined by rings of 12 oxygen atoms. Typically, the minimum dimensions of these openings are between 7 and 8 Å.

The majority of thiopyrylium cations are too bulky to be adsorbed inside the large-pore zeolite cavities from the outer to the inner areas of the particles. However, those cations with molecular dimensions of less than 13 Å can fit inside the zeolite cavities.

To this end, it is necessary to develop a synthesis process of the type of those commonly named boat in a bottle types, which are based on the adsorption of suitable precursors of smaller molecular size and which can spread to the inside of the intracrystalline and inner spaces of the zeolites. Once inside, a chemical reaction occurs, causing the formation of the desired product. Once formed, this compound remains immobilised inside the cavities, since the dimensions thereof do not allow it to pass through the cavity openings.

This strategy is sufficiently documented in the literature, as disclosed in J. C. Escaiano & H. Garcia, "Intrazeolite photochemistry: toward supramolecular control of molecular photochemistry", Accounts of Chemical Research, 1999, 32 783-793*,* for the formation of other organic cations, including the 2,4,6-triphenylpyrylium cation and tritylium. However, it has not been applied to the thiopyrylium cation nor to any of its derivatives. Therefore, the material described in the present report has not as yet been prepared.

Neither has the reaction used here for the synthesis of the thiopyrylium cations been applied prior to the preparation of any material containing an organic cation in a zeolitic material.

In the case of mesoporous aluminosilicates with a hexagonal channel structure (MCM-41) or cubic channel structure (MCM-48), these are materials where the walls do not have a crystalline structure, and are therefore amorphous materials. However, their structure defines hexagonal channels in one direction of space (MCM-41) or in the three directions (MCM-48), the size distribution of which is extremely regular, as with the zeolites. In the case of porous aluminosilicates, it is possible to control the dimensions of the channels at will by means of material preparation conditions between 20 and 100 Å.

### BRIEF DESCRIPTION OF THE INVENTION

The object of the present invention is a material comprising an organic cation trapped in a support, characterised in that said cation is selected among a thiopyrylium cation, a thiopyrylium cation derivative and mixtures thereof, and said cation is trapped in a zeolitic material, such as zeolite or a structured porous aluminosilicate.

A further object of the present invention is a process for preparing a material comprising a cation selected among a thiopyrylium cation, a thiopyrylium cation derivative and mixtures thereof, trapped in a zeolitic material such as a zeolite or a structured porous aluminosilicate, characterised in that it comprises carrying out direct adsorption of the cation from solution or a boat in a bottle synthesis.

### DESCRIPTION OF THE INVENTION

The present invention describes a material comprising an organic cation trapped in a support, characterised in that said cation is selected among a thiopyrylium cation, a thiopyrylium cation derivative and mixtures thereof, and said cation is trapped in a zeolitic material which is a large pore zeolite or a mesoporous MCM-41 or MCM-48 aluminosilicate having a pore size between 20 and 100Å.

The thiopyrylium cation and derivatives correspond to the formula: 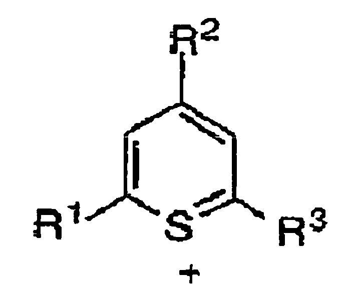 where R¹, R² and R³ can be equal or different and can be any combination of H, alkyl groups (CH₃, CH₃CH₂, propyl, *i*-Pr, etc.) and aryl (phenyl and substituted phenyls, naphthyls and substituted naphthyls, heterocycles), etc.

Preferably, the cation is 2,4,6-triphenylthiopyrylium. Even more preferably, said cation is 2,4,6-triphenylthiopyrylium and is trapped in Y zeolite or Beta zeolite, in ratios by weight of the cation ranging between 0.1 and 10%.

Zeolites, and particularly the Y zeolite and the Beta zeolite, can comprise heteroatoms in the lattice. One preferred heteroatom is titanium.

Alternatively, the material in which the cation is trapped is an MCM-41 or MCM-48 structured porous aluminosilicate. In particular, the aluminosilicate can also comprise heteroatoms. Preferred heteroatoms are titanium atoms.

The content of thiopyrylium ions trapped in the zeolitic material ranges at will between 1 and 15% by weight of the cation in the case where the zeolitic material is large-pore zeolites, and up to 50% by weight of the cation in the case where the zeolitic material is structured porous aluminosilicates. The ratio by weight of the organic cation is regulated by suitably varying the amount of reagent precursors. The amount of thiopyrylium cation contained in the material can be experimentally determined by elemental combustion analysis (C and S) and by thermogravimetry. Differential scanning calorimetry under air current shows that the organic cation remains unaltered up to temperatures of 400°C, a progressive degradation occurring at higher temperatures. The peak at which the released heat curve maximum occurs is around 550°C for the 2,4,6- triphenylthiopyrylium sample in Y zeolite.

The UV-Vis diffuse reflectance spectra of these materials are in accordance with the UV-Vis absorption spectra for these same inorganic cations in acetonitrile, dichloromethane and other organic solvents. Only small solvatochromic shifts can be seen in the wavelength absorption maximums of less than 40 nm.

The IR spectra of the thiopyrylium cations trapped in large-pore zeolites and aluminosilicates also coincide with the IR spectra of the tetrafluoroborate salts of the same cations in KBr pellets in those areas where the aluminosilicate lattice does not interfere. The obtainment of these IR spectra is carried out at room temperature, but requires the prior removal of the coadsorbed water in the samples by means of heating under vacuum conditions of 10⁻² Pa at 200°C for 1 hour.

The thiopyrylium samples incorporated in large-pore zeolites or in mesoporous aluminosilicates show emission after being stimulated at λₘₐₓ of the UV-Vis absorption band. In this case the room temperature emission spectra correspond to a combination of fluorescence and phosphorescence, in which the latter generally predominates.

A further object of the present invention is a process for preparing a material comprising at least one thiopyrylium cation or a thiopyrylium cation derivative, or mixtures thereof, trapped in a zeolitic material such as a zeolite or a structured porous aluminosilicate, characterised in that it comprises a direct adsorption of the cation from solution or a boat in a bottle synthesis.

Below, diagram 1 shows a boat in a bottle synthesis according to said process: 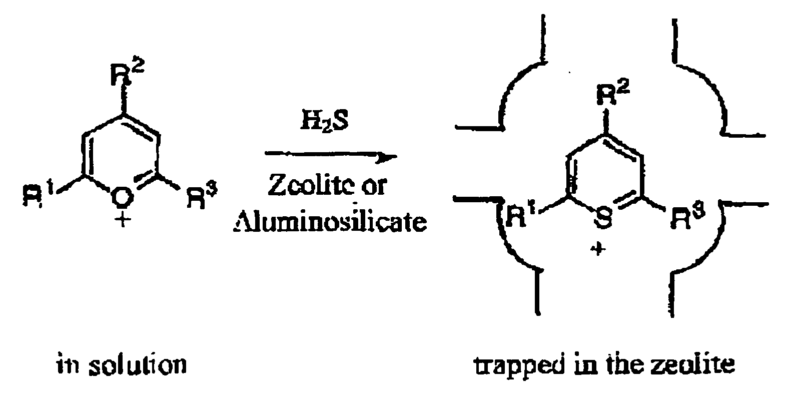 where R¹, R² and R³ have the previously indicated meaning.

The preparation process of the material of the present invention includes:
- a first step wherein the zeolitic material is dehydrated, for example, by heating at a temperature of 500°C for 1 hour or more.
- a second step wherein the dehydrated zeolitic material is poured, still hot, in an organic solution saturated with hydrogen sulfide or bisulfide, and a first suspension is obtained.
- a third step wherein said first suspension is maintained in a hydrogen sulfide or bisulfide atmosphere and is magnetically or mechanically stirred for at least 30 minutes with a view to making the solid become saturated with hydrogen sulfide or bisulfide before proceeding with the preparation.
- a fourth step wherein the suitable amount of at least one corresponding pyrylium compound precursor of a thiopyrylium cation is added to said suspension. To this end, it is preferable for the required amount of pyrylium compound precursor to dissolve in the reaction solvent prior to its addition to the suspension of zeolite or aluminosilicate saturated with hydrogen sulfide. The amount of compound precursor, at a ratio with respect to the zeolite, ranges between 1 and 15%, and between 1 and 50% by weight for mesoporous aluminosilicates.
- a fifth step wherein the suspension is maintained at a temperature between room temperature and that of the reflux of the solvent in a hydrogen sulfide or bisulfide atmosphere, for a sufficient length of time.
- a sixth step wherein, once this time is elapsed, the solid contained in said suspension is filtered, washed with fresh solvent and thoroughly extracted with an organic solvent until the solvent no longer contains remains of the pyrylium compound precursor or any of the products derived therefrom, and
- a seventh step wherein said solid is evacuated at reduced pressure and is stored protected from the light and from the atmosphere.

When the zeolitic material is a zeolite, any large-pore sized zeolite, such as Y and Beta, and whatever its Si/Al ratio, charge compensation cations, particle size, surface area and crystallinity may be, can be used.

In the case where the zeolitic material is a structured porous aluminosilicate, this can be of the MCM-41 hexagonal or MCM-48 cubic type, with any Si/Al ratio, particle size, surface area and mesopore size between 20 and 100 Å.

The organic solvent used can be alkane, cycloalkane, halogenated compounds, acetonitrile or any other in general which dissolves the pyrylium salt precursor. The volume of solvent used can range between 1 to 20% by weight. A convenient manner of saturating the solvent with hydrogen sulfide is to slowly bubble this gas from a cylinder containing it under pressure for a sufficient length of time.

In the case of structured porous aluminosilicates with a pore size between 20 and 100 Å, an alternative preparation method to the one previously described is also possible, and consists of the direct incorporation of thiopyrylium salts from an organic solution towards the inner area of the aluminosilicate pores.

The following figures and example show some aspects of the invention.

### BRIEF DESCRIPTION OF THE FIGURES

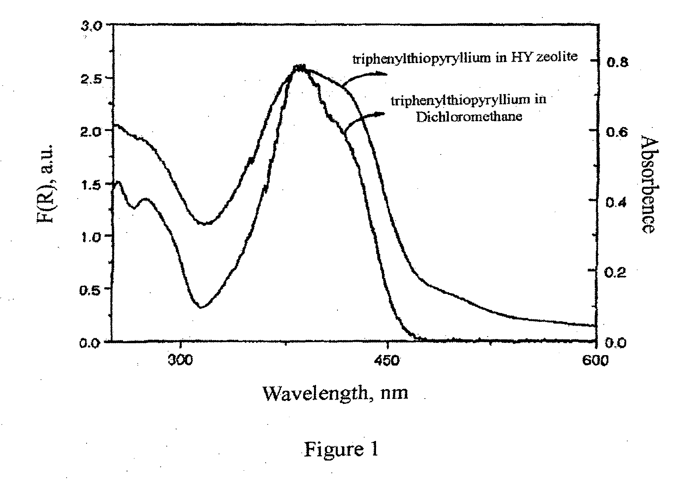

Figure 1 shows a UV-Vis diffuse reflectance spectrum for the case of the 2,4,6-triphenylthiopyrylium cation in Y zeolite.

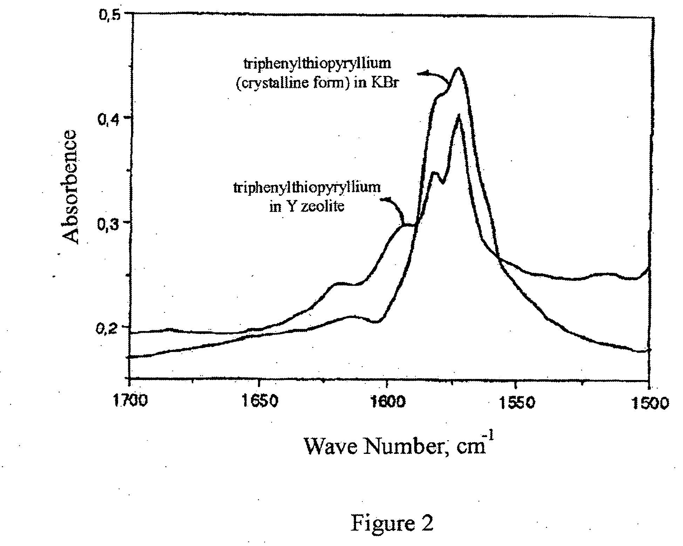

Figure 2 shows a typical IR spectrum of the 2,4,6-triphenylthiopyrylium cation trapped in a zeolite and its comparison with that of the tetrafluoroborate salt of the same thiopyrylium cation.

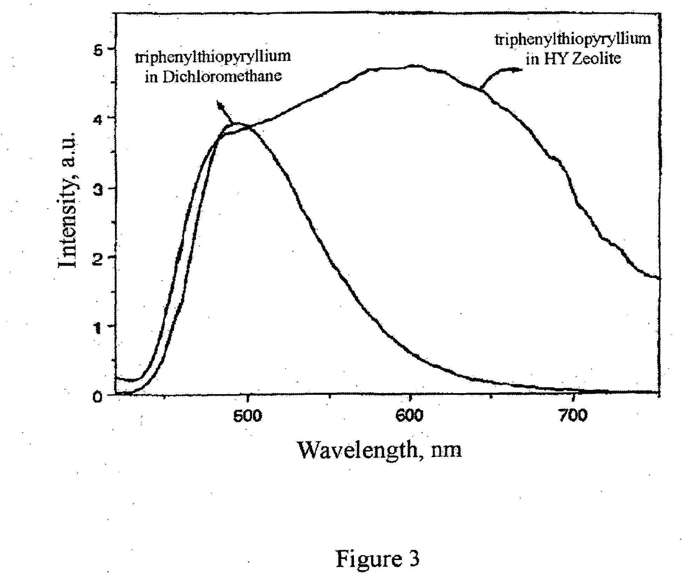

Figure 3 shows a typical emission spectrum after being stimulated at λₘₐₓ of the UV-Vis absorption band, of the 2,4,6-triphenylthiopyrylium cation trapped in Y zeolite.

### Example

In a preferred embodiment of the materials of the present invention, 10 grams of Y zeolite (Si/Al 2.4) are used in their sodium form and with an average particle size of 1 µm. This material is dehydrated overnight in an oven under air atmosphere at 500°C. After its dehydration, the zeolite is left to cool to 150°C and is carefully poured onto a 100 ml cyclohexane solution which has been saturated with hydrogen sulfide. The suspension is stirred for 30 minutes in a hydrogen sulfide atmosphere. Then, 10 ml of cyclohexane containing 0.8 g of 2,4,6-triphenylpyrylium tetrafluoroborate are added, and the suspension is stirred for 1 day at reflux temperature of the cyclohexane (68°C at the pressure of an atmosphere). After this time has elapsed, the suspension is filtered and the solid is thoroughly extracted with dichloromethane in Soxhlet equipment for one day. The remains of the solvent of the extraction are vacuum removed before proceeding to the spectroscopic characterisation of those materials and to their use as photocatalysts.

## Claims

1. A material comprising an organic cation trapped in a support, **characterized in that** said organic cation is at least one of a thiopyrylium cation and a thiopyrylium cation derivative corresponding to the formula: 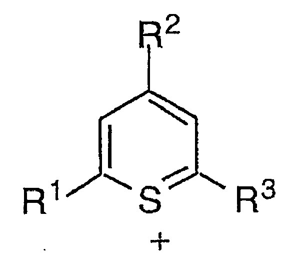 wherein R¹, R² and R³ are equal or different groups selected among H, alkyl groups, aryl groups and combinations thereof, and **in that** the support is a large pore zeolite or a mesoporous MCM-41 or MCM-48 aluminosilicate having a pore size between 20 and 100 Å.

2. A material according to claim 1 , **characterised in that** the cation is trapped in an amount comprised between 1 and 15% by weight.

3. A material according to claim 1, **characterised in that** the meso porous aluminosilicate contains trapped cation in an amount comprised between 1 and 50% by weight.

4. A material according to claim 1, **characterised in that** the alkyl groups are selected among methyl, ethyl, propyl and iso-propyl.

5. A material according to claim 1, **characterised in that** the aryl groups are selected among phenyl, substituted phenyl, naphthyl, substituted naphthyl, and heterocycle.

6. A material according to claim 1, **characterised in that** the cation is 2,4,6-triphenylthiopyrylium.

7. A material according to claim 1, **characterised in that** the zeolite is Y zeolite.

8. A material according to claim 1, 6 or 7, **characterised in that** the cation is 2,4,6-triphenylthiopyrylium and is trapped in Y zeolite.

9. A material according to claim 8, **characterised in that** the 2,4,6-triphenylthiopyrylium cation is trapped in Y zeolite in ratios by weight of the cation ranging between 0.1 and 10%.

10. A material according to claim 1, **characterised in that** the zeolite is Beta zeolite.

11. A material according to claim 1 or 10, **characterised in that** the Beta zeolite contains the trapped 2,4,6-triphenylthiopyrylium cation.

12. A material according to claim 11, **characterised in that** the 2,4,6-triphenylthiopyrylium cation is present in ratios by weight of cation ranging between 0.1 and 10%.

13. A material according to claim 1 or 10, **characterised in that** the zeolite is Beta zeolite comprising heteroatoms in the lattice.

14. A material according to claim 13, **characterised in that** the heteroatoms are titanium atoms.

15. A material according to claim 1, **characterised in that** MCM-41 or MCM-48 meso porous aluminosilicate contains the trapped 2,4,6-triphenylthiopyrylium cation.

16. A material according to claim 15, **characterised in that** the meso porous aluminosilicate also comprises heteroatoms.

17. A material according to claim 16, **characterised in that** the heteroatoms are titanium atoms.

18. A process for preparing a material according to any one of claims 1-17, **characterised in that** it comprises:
- a first step wherein the support is dehydrated,
- a second step wherein the dehydrated support is poured, still hot, in an organic solution saturated with hydrogen sulfide or bisulfide, and a first suspension is obtained,
- a third step wherein said first suspension is maintained in a hydrogen sulfide or bisulfide atmosphere and is magnetically or mechanically stirred for at least 30 minutes,
- a fourth step wherein the suitable amount of at least one corresponding pyrylium compound precursor of a thiopyrylium compound is added to said suspension,
- a fifth step wherein said suspension is maintained at a temperature between room temperature and the reflux temperature of the solvent in a hydrogen sulfide or bisulfide atmosphere,
- a sixth step wherein the solid contained in said suspension is filtered, washed with fresh solvent and thoroughly extracted with an organic solvent, and
- a seventh step wherein said solid is evacuated at reduced pressure and is stored protected from the light and from the atmosphere.

19. A process according to claim 18 **characterised in that** in the first step the support is dehydrated by heating at a temperature of 500 °C for at least 1 hour.

20. A process according to claim 18, **characterised in that** in the fourth step the required amount of pyrylium compound precursor is dissolved in an organic solvent which is the same as that of the second step, prior to its addition to the suspension of zeolitic material saturated with hydrogen sulfide or bisulfide.

21. A process according to claim 18, **characterised in that** the support is a zeolite and the amount of pyrylium compound precursor ranges in a ratio between 1 to 15% by weight with respect to the zeolite.

22. A process according to claim 18, **characterised in that** the support is a meso porous aluminosilicate and the amount of pyrylium compound precursor ranges in a ratio between 1 and 50% by weight with respect to said meso porous aluminosilicate.

23. A process according to claim 18, **characterised in that** in the sixth step the extraction is carried out until the solvent no longer contains remains of the pyrylium compound precursor or any other product derived therefrom.

24. A process according to claim 18, **characterised in that** in the second step an organic solvent is used, selected among alkane, cycloalkane, halogenated compounds and acetonitrile.

25. A process according to claim 18, **characterised in that** in the second step a volume of solvent is used comprised between 1 to 20% by weight.

26. A process according to claim 18, **characterised in that** hydrogen sulfide is used and the thiopyrylium cation is 2,4,6-triphenylthiopyrylium.

27. A process according to claim 18, **characterised in that** the support is a porous aluminosilicate with a pore size exceeding 100 Å and a direct adsorption of the cation is carried out from solution.

## Patentansprüche

1. Material, umfassend ein organisches Kation, eingeschlossen in einen Träger, **dadurch gekennzeichnet, daß** das organische Kation zumindest eines eines Thiopyryliumkations und eines Thiopyryliumkationderivats ist, das der Formel: 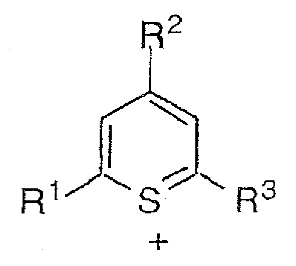 entspricht,
worin R¹, R² und R³ gleiche oder verschiedene Gruppen sind, ausgewählt aus H, Alkylgruppen, Arylgruppen und Kombinationen davon, und daß der Träger ein großporiger Zeolith oder ein mesoporöses MCM-41- oder MCM-48-Alumosilicat mit einer Porengröße zwischen 20 und 100 Å ist.

2. Material nach Anspruch 1, **dadurch gekennzeichnet, daß** das Kation in einer Menge zwischen 1 und 15 Gew.-% eingeschlossen ist.

3. Material nach Anspruch 1, **dadurch gekennzeichnet, daß** das mesoporöse Alumosilicat das eingeschlossene Kation in einer Menge zwischen 1 und 50 Gew.-% enthält.

4. Material nach Anspruch 1, **dadurch gekennzeichnet, daß** die Alkylgruppen aus Methyl, Ethyl, Propyl und Isopropyl ausgewählt sind.

5. Material nach Anspruch 1, **dadurch gekennzeichnet, daß** die Arylgruppen aus Phenyl, substituiertem Phenyl, Naphthyl, substituiertem Naphthyl und einem Heterocyclus ausgewählt sind.

6. Material nach Anspruch 1, **dadurch gekennzeichnet, daß** das Kation 2,4,6-Triphenylthiopyrylium ist.

7. Material nach Anspruch 1, **dadurch gekennzeichnet, daß** der Zeolith Y-Zeolith ist.

8. Material nach Anspruch 1 6 oder 7, **dadurch gekennzeichnet, daß** das Kation 2,4,6-Triphenylthiopyrylium ist und in Y-Zeolith eingeschlossen ist.

9. Material nach Anspruch 8, **dadurch gekennzeichnet, daß** das 2,4,6-Triphenylthiopyryliumkation in Y-Zeolith in Gewichtsverhältnissen des Kations im Bereich von 0,1 bis 10 % eingeschlossen ist.

10. Material nach Anspruch 1, **dadurch gekennzeichnet, daß** der Zeolith Beta-Zeolith ist.

11. Material nach Anspruch 1 oder 10, **dadurch gekennzeichnet, daß** der Beta-Zeolith das eingeschlossene 2,4,6-Triphenylthiopyryliumkation enthält.

12. Material nach Anspruch 11, **dadurch gekennzeichnet, daß** das 2,4,6-Triphenylthiopyryliumkation in Gewichtsverhältnissen des Kations im Bereich von 0,1 bis 10 % vorliegt.

13. Material nach Anspruch 1 oder 10, **dadurch gekennzeichnet, daß** der Zeolith Beta-Zeolith, umfassend Heteroatome im Gitter, ist.

14. Material nach Anspruch 13, **dadurch gekennzeichnet, daß** die Heteroatome Titanatome sind.

15. Material nach Anspruch 1, **dadurch gekennzeichnet, daß** das mesoporöse MCM-41- oder MCM-48-Alumosilicat das eingeschlossene 2,4,6-Triphenylthiopyryliumkation enthält.

16. Material nach Anspruch 15, **dadurch gekennzeichnet, daß** das mesoporöse Alumosilicat ferner Heteroatome umfaßt.

17. Material nach Anspruch 16, **dadurch gekennzeichnet, daß** die Heteroatome Titanatome sind.

18. Verfahren zur Herstellung eines Materials nach einem der Ansprüche 1 bis 17, **dadurch gekennzeichnet, daß** es:
- einen ersten Schritte, worin der Träger dehydratisiert wird,
- einen zweiten Schritte, worin der dehydratisierte Träger noch heiß in eine organische mit Hydrogensulfid oder Bisulfid gesättigte Lösung gegossen wird und eine erste Suspension erhalten wird,
- einen dritten Schritte, worin die erste Suspension in einer Hydrogensulfid- oder Bisulfid-Atmosphäre gehalten und magnetisch oder mechanisch für mindestens 30 Minuten gerührt wird,
- einen vierten Schritte, worin die geeignete Menge zumindest eines entsprechenden Pyryliumverbindungspräkursors einer Thiopyryliumverbindung zu der Suspension zugegeben wird,
- einen fünften Schritte, worin die Suspension bei einer Temperatur zwischen Raumtemperatur und der Rückflußtemperatur des Lösungsmittels in einer Hydrogensulfid- oder Bisulfid-Atmosphäre gehalten wird,
- einen sechsten Schritte, worin der in der Suspension enthaltene Feststoff filtriert, mit frischem Lösungsmittel gewaschen und mit einem organischen Lösungsmitte gründlich extrahiert wird, und
- einen siebenten Schritte, worin der Feststoff bei reduziertem Druck evakuiert und geschützt vor Licht und Luft gelagert wird, umfaßt.

19. Verfahren nach Anspruch 18, **dadurch gekennzeichnet, daß** der Träger im ersten Schritt durch Erhitzen bei einer Temperatur von 500 °C für mindestens 1 Stunde dehydratisiert wird.

20. Verfahren nach Anspruch 18, **dadurch gekennzeichnet, daß** die erforderliche Menge an Pyryliumverbindungspräkursor im vierten Schritt in einem organischen Lösungsmittel, welches dasselbe wie das aus dem zweiten Schritt ist, gelöst wird bevor es zu der Suspension des mit Hydrogensulfid oder Bisulfid gesättigten zeolithischen Materials zugegeben wird.

21. Verfahren nach Anspruch 18, **dadurch gekennzeichnet, daß** der Träger ein Zeolith ist und die Menge des Pyryliumverbindungspräkursors in einem Anteil von 1 bis 15 Gew.-%, bezogen auf den Zeolith, vorliegt.

22. Verfahren nach Anspruch 18, **dadurch gekennzeichnet, daß** der Träger ein mesoporöses Alumosilicat ist und die Menge des Pyryliumverbindungspräkursors in einem Anteil von 1 bis 50 Gew.-%, bezogen auf das mesoporöse Alumosilicat, vorliegt.

23. Verfahren nach Anspruch 18, **dadurch gekennzeichnet, daß** die Extraktion im sechsten Schritt so lange durchgeführt wird, bis das Lösungsmittel keine Rückstände des Pyryliumverbindungspräkursors oder irgendeines anderen daraus stammenden Restes enthält.

24. Verfahren nach Anspruch 18, **dadurch gekennzeichnet, daß** im zweiten Schritt ein organisches Lösungsmittel verwendet wird, ausgewählt aus Alkan, Cycloalkan, halogenierten Verbindungen und Acetonitril.

25. Verfahren nach Anspruch 18, **dadurch gekennzeichnet, daß** im zweiten Schritt ein Lösungsmittelvolumen zwischen 1 und 20 Gew.-% verwendet wird.

26. Verfahren nach Anspruch 18, **dadurch gekennzeichnet, daß** Hydrogensulfid verwendet wird und das Thiopyryliumkation 2,4,6-Triphenylthiopyrylium ist.

27. Verfahren nach Anspruch 18, **dadurch gekennzeichnet, daß** der Träger ein poröses Alumosilicat mit einer Porengröße über 100 Å ist und eine direkte Adsorption des Kations aus der Lösung durchgeführt wird.

## Revendications

1. Matériau comprenant un cation organique piégé dans un support, **caractérisé en ce que** ledit cation organique est au moins un parmi un cation thiopyrylium et un dérivé de cation thiopyrylium correspondant à la formule : 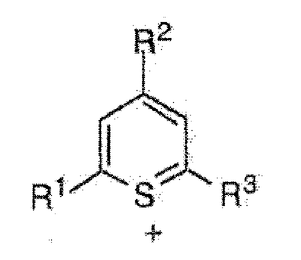 dans laquelle R¹, R² et R³ sont des groupes identiques ou différents choisis parmi H, les groupes alkyles, les groupes aryles et leurs combinaisons, et **en ce que** le support est une zéolithe à larges pores ou un aluminosilicate mésoporeux MCM-41 ou MCM-48 ayant une taille de pores comprise entre 20 et 100 Å.

2. Matériau selon la revendication 1, **caractérisé en ce que** le cation est piégé dans une quantité comprise entre 1 et 15 % en poids.

3. Matériau selon la revendication 1, **caractérisé en ce que** l'aluminosilicate mésoporeux contient un cation piégé dans une quantité comprise entre 1 et 50 % en poids.

4. Matériau selon la revendication 1, **caractérisé en ce que** les groupes alkyles sont choisis parmi méthyle, éthyle, propyle et isopropyle.

5. Matériau selon la revendication 1, **caractérisé en ce que** les groupes aryles sont choisis parmi phényle, phényle substitué, naphthyle, naphtyle substitué et hétérocycle.

6. Matériau selon la revendication 1, **caractérisé en ce que** le cation est le 2,4,6-triphénylthiopyrylium.

7. Matériau selon la revendication 1, **caractérisé en ce que** la zéolithe est une zéolithe Y.

8. Matériau selon la revendication 1, 6 ou 7, **caractérisé en ce que** le cation est le 2,4,6-triphénylthiopyrylium et est piégé dans la zéolithe Y.

9. Matériau selon la revendication 8, **caractérisé en ce que** le cation 2,4,6-triphénylthiopyrylium est piégé dans la zéolithe Y avec des rapports en poids du cation allant de 0,1 et 10 %.

10. Matériau selon la revendication 1, **caractérisé en ce que** la zéolithe est une zéolithe béta.

11. Matériau selon la revendication 1 ou 10, **caractérisé en ce que** la zéolithe béta contient le cation piégé 2,4,6-triphénylthiopyrylium.

12. Matériau selon la revendication 11, **caractérisé en ce que** le cation 2,4,6-triphénylthiopyrylium est présent dans des rapports en poids du cation compris entre 0,1 et 10 %.

13. Matériau selon la revendication 1 ou 10, **caractérisé en ce que** la zéolithe est une zéolithe béta comprenant des hétéroatomes dans le réseau.

14. Matériau selon la revendication 13, **caractérisé en ce que** les hétéroatomes sont des atomes de titane.

15. Matériau selon la revendication 1, **caractérisé en ce que** l'aluminosilicate mésoporeux MCM-41 ou MCM-48 contient le cation piégé 2,4,6-triphénylthiopyrylium.

16. Matériau selon la revendication 15, **caractérisé en ce que** l'aluminosilicate mésoporeux comprend également des hétéroatomes.

17. Matériau selon la revendication 16, **caractérisé en ce que** les hétéroatomes sont des atomes de titane.

18. Procédé de préparation d'un matériau selon l'une quelconque des revendications de 1 à 17, **caractérisé en ce qu'**il comprend :
- une première étape dans laquelle le support est déshydraté,
- une deuxième étape dans laquelle le support déshydraté est versé, encore chaud, dans une solution organique saturée avec du sulfure ou du disulfure d'hydrogène, et une première suspension est obtenue,
- une troisième étape dans laquelle ladite première suspension est maintenue dans une atmosphère de sulfure ou de disulfure d'hydrogène et est soumise à une agitation magnétique ou mécanique pendant au moins 30 minutes,
- une quatrième étape dans laquelle la quantité appropriée d'au moins un précurseur de composé de pyrylium correspondant d'un composé de thiopyrylium, est ajoutée à ladite suspension,
- une cinquième étape dans laquelle ladite suspension est maintenue à une température dont la valeur est comprise entre la température ambiante et la température de reflux du solvant dans une atmosphère de sulfure ou de disulfure d'hydrogène,
- une sixième étape dans laquelle le solide contenu dans ladite suspension est filtré, lavé avec du solvant frais et entièrement extrait avec un solvant organique, et
- une septième étape dans laquelle ledit solide est évacué à pression réduite et est stocké à l'abri de la lumière et de l'atmosphère.

19. Procédé selon la revendication 18, **caractérisé en ce que** dans la première étape le support est déshydraté en étant chauffé à une température de 500°C pendant au moins 1 heure.

20. Procédé selon la revendication 18, **caractérisé en ce que** dans la quatrième étape la quantité requise de précurseur de composé de pyrylium est dissoute dans un solvant organique qui est le même que celui de la deuxième étape, avant son addition à la suspension de matériau zéolithique saturé avec du sulfure ou du disulfure d'hydrogène.

21. Procédé selon la revendication 18, **caractérisé en ce que** le support est une zéolithe et la quantité de précurseur du composé de pyrylium se situe dans un rapport entre 1 et 15 % en poids par rapport à la zéolithe.

22. Procédé selon la revendication 18, **caractérisé en ce que** le support est un aluminosilicate mésoporeux et la quantité de précurseur du composé de pyrylium se situe dans un rapport entre 1 et 50 % en poids par rapport audit aluminosilicate mésoporeux.

23. Procédé selon la revendication 18, **caractérisé en ce que** dans la sixième étape, l'extraction est effectuée jusqu'à ce que le solvant ne contienne plus de restes du précurseur de composé de pyrylium ou de tout autre produit qui en dérive.

24. Procédé selon la revendication 18, **caractérisé en ce que** dans la deuxième étape un solvant organique choisi parmi les alcanes, les cycloalcanes, les composés halogénés et l'acétonitrile est utilisé.

25. Procédé selon la revendication 18, **caractérisé en ce que** dans la deuxième étape un volume de solvant compris entre 1 et 20 % en poids est utilisé.

26. Procédé selon la revendication 18, **caractérisé en ce que** du sulfure d'hydrogène est utilisé et que le cation thiopyrylium est le 2,4,6-triphénylthiopyrylium.

27. Procédé selon la revendication 18, **caractérisé en ce que** le support est un aluminosilicate poreux avec une taille de pores supérieure à 100 Å et qu'une adsorption directe du cation est effectuée à partir d'une solution.
